# FASCICULE DE BREVET EUROPEEN

(11) **EP 0 605 264 B1**
(45) Date de publication et mention de la délivrance du brevet: **20.05.1998**
(21) Numéro de dépôt: 93402862.2
(22) Date de dépôt: 26.11.1993
(51) Int. Cl.: A61N 1/37, A61N 1/368

(54) **Stimulateur cardiaque implantable**
Implantierbarer Herzschittmacher
Implantable pacemaker

(30) Priorité: 23.12.1992 FR 9215661
(43) Date de publication de la demande: 06.07.1994
(73) Titulaire: ELA MEDICAL (Société anonyme), F-92541 Montrouge Cédex (FR)
(72) Inventeur: Lee, Chik-Yam, F-94110 Arcueil (FR); Claudon, Laurent, F-54180 Heillecourt (FR); Dal Molin, Renzo, F-92220 Bagneux (FR)
(74) Mandataire: Laget, Jean-Loup

(56) Documents cités:
- WO-A-91/10472
- DE-A- 3 226 345
- US-A- 4 596 252
- US-A- 4 708 144
- US-A- 4 974 589
- US-A- 5 024 221

## Description

L'invention concerne un stimulateur cardiaque implantable.

La plupart des stimulateurs cardiaques fonctionnent selon le mode inhibé : une impulsion de stimulation n'est délivrée que si aucune activité spontanée n'est détectée pendant une période d'écoute correspondant à la durée d'un cycle cardiaque (800 ms pour un rythme normal de 75 cpm). Ce mode de fonctionnement permet une économie d'énergie, c'est-à-dire un accroissement de la longévité du stimulateur. Il permet également, du point de vue physiologique, d'éviter une concurrence entre le rythme spontané du patient et la fréquence de stimulation du stimulateur cardiaque, comme c'est le cas en mode asynchrone.

Dans un stimulateur cardiaque l'activité cardiaque, représentée par une variation de potentiel des cellules du myocarde, est recueillie au moyen de sondes placées sur les parois de l'oreillette et ou du ventricule suivant le type du stimulateur cardiaque (AAI, VVI, DDD).

Certains problèmes liés au recueil proviennent des perturbations engendrées par le stimulateur lui-même. En effet, les impulsions de stimulation provoquent d'importantes variations du signal, aussi bien dans la chambre stimulée que dans la chambre non stimulée. Pour cela, il est nécessaire de prévoir des solutions afin de ne pas confondre une déflexion due à une stimulation avec une déflexion due à un événement spontané.

Le recueil d'une impulsion de stimulation dans la chambre non stimulée pose surtout un problème lors d'un recueil ventriculaire après une stimulation auriculaire. En effet, il est nécessaire d'écouter le ventricule le plus rapidement possible après la stimulation afin de vérifier la bonne conduction auriculo-ventriculaire.

Le document DE 32 26 345 décrit, dans un stimulateur cardiaque implantable, l'utilisation d'un convertisseur analogique-numérique à modulation delta dans la chaîne de recueil de l'activité cardiaque.

Le document WO 91/10472 décrit une méthode pour s'affranchir des phénomènes transitoires dans un amplificateur du signal ventriculaire, à capacités commutées après la délivrance d'une impulsion de stimulation dans l'oreillette. Cette méthode consiste à faire varier la fréquence de l'horloge qui contrôle la commutation des capacités.

Le stimulateur cardiaque implantable selon l'invention, recueille les variations de potentiel engendrées par l'activité cardiaque spontanée. Il est également susceptible de transmettre par télémétrie, vers l'extérieur, l'électrocardiogramme (ECG) endocavitaire.

Un premier but de l'invention est de proposer un stimulateur cardiaque implantable qui utilise la même chaîne électronique pour la transmission de l'ECG vers l'extérieur, et pour la recherche de l'activité spontanée du coeur.

Un autre but de l'invention est de proposer un stimulateur cardiaque implantable, capable de suivre le plus fidèlement possible une déflexion due à une stimulation, afin de mesurer correctement le paramètre d'efficacité, ou d'inefficacité, de la stimulation, en vue de faire fonctionner un algorithme d'adaptation de l'énergie de stimulation.

Un autre but encore de l'invention est de proposer un stimulateur cardiaque implantable comportant un réglage automatique de la sensibilité afin de prendre en compte l'évolution dans le temps de la forme du signal recueilli.

Un autre but de l'invention est de prévoir un ajustage automatique de la période de "blanking" en fonction de l'importance de la diaphonie présente sur le signal.

La présente invention a pour objet un stimulateur cardiaque implantable, dans lequel la chaîne électronique d'acquisition et de détection de l'activité cardiaque endocavitaire comporte un filtre passif, un amplificateur à capacités commutées, un convertisseur analogique-numérique, un microprocesseur, et un algorithme pour assurer la détection des événements cardiaques spontanés, caractérisé en ce que :
- le filtre présente une bande passante s'étendant de 0,1 à 80 Hz environ ;
- l'amplificateur présente à la sortie une dynamique de 0,8 V environ et un gain inférieur à 50 ;
- le convertisseur analogique-numérique est à modulation delta, à fréquence et à pas variables et adaptés en fonction du signal à convertir.

Selon d'autres caractéristiques preferées :
- le convertisseur anologique-numérique fonctionne à deux fréquences, l'une d'environ 1 kHz, l'autre d'environ 4 kHz;
- la chaîne électronique d'acquisition et de détection de l'activité cardiaque présente une bande passante large, un gain faible et une dynamique grande pour assurer la détection des événements spontanés auriculaires et/ou ventriculaires, la mesure du paramètre d'efficacité de stimulation, et la transmission d'un ECG endocavitaire par télémétrie ;
- un réglage automatique de la période de "blanking" ;
- un réglage automatique de la sensibilité ;
- la commande de changement de fréquence et de changement de pas du convertisseur analogique-numérique est réalisée par comparaison de la valeur absolue de la variation d'amplitude du signal recueilli pendant une durée de 6 à 12 ms et de préférence de 9 ms avec un seuil dénommé seuil d'accélération ;
- la détection d'un événement cardiaque spontané auriculaire ou ventriculaire est réalisée par une comparaison de la valeur absolue de la variation d'amplitude du signal recueilli pendant une durée de 6 à 12 ms et de préférence de 9 ms avec un seuil appelé seuil de détection ;
- la fonction de détection d'un événement cardiaque auriculaire ou ventriculaire est réalisée par traitement numérique avec un microcontrôleur dédié ;
- la fonction de détection d'un événement cardiaque auriculaire ou ventriculaire est réalisée par traitement numérique avec un microcontrôleur dédié ;
- le signe d'une variation d'amplitude du signal recueilli pendant une durée de 6 à 12 ms et de préférence de 9 ms avant la fin de la période réfractaire absolue (PRA) supérieure au seuil de détection étant mémorisé, la détection, après la PRA, d'un événement spontané n'est indiquée que si la valeur absolue de la variation d'amplitude du signal recueilli pendant une durée de 6 à 12 ms et de préférence de 9 ms est supérieure au seuil de détection et si le signe de cette variation est contraire à celui mémorisé durant la PRA ;
- la durée du "blanking" ventriculaire est réglable manuellement ou automatiquement, et dans le cas d'un réglage automatique, la durée de "blanking" est ajustée à chaque stimulation auriculaire en fonction de l'amplitude de la diaphonie ;
- au cours d'un réglage automatique de la durée du "blanking", la fin de celui-ci est obtenue lorsque le signe s'inverse entre deux variations consécutives du signal recueilli pendant une durée de 6 à 12 ms et de préférence de 9 ms ;
- l'amplitude de la déflexion du signal ventriculaire consécutive à une stimulation auriculaire est mesurée à la fin de la période de "blanking" et le signe de cette déflexion est mémorisé, et si l'amplitude de cette déflexion est supérieure au seuil de détection, à l'issue de la période de "blanking" seules les valeurs absolues des variations d'amplitude du signal ventriculaire recueilli pendant une durée de 6 à 12 ms et de préférence de 9 ms supérieures au seuil de détection et dont le signe est identique à celui de la déflexion seront considérées comme étant dues à la présence d'un événement ventriculaire, et dans le cas contraire, la détection est réalisée sans contrainte de signe ;
- lors d'un réglage automatique de sensibilité, une recherche de la valeur absolue de la variation d'amplitude maximale du signal recueilli pendant une durée de 6 à 12 ms et de préférence de 9 ms débute sur une détection d'un événement cardiaque spontané auriculaire et/ou ventriculaire et se prolonge durant une période égale de préférence à 48 ms ;
- lors d'un réglage automatique de la sensibilité, la sensibilité initiale est programmable et cette valeur multipliée de préférence par 0.375, remplace en cas de stimulation la variation d'amplitude maximale recherchée après une détection selon la revendication 13 ;
- lors d'un réglage automatique de la sensibilité le nouveau seuil de détection est fixé à un certain pourcentage, de préférence 37,5%, de la moyenne des huit dernières valeurs maximales de la variation du signal sur 9 ms recueilli à chaque détection et stimulation ;

D'autres caractéristiques ressortent de la description qui suit faite avec référence au dessin annexé sur lequel on peut voir :
Figure 1 : un schéma simplifié de la chaîne électronique d'acquisition de l'activité cardiaque dans un stimulateur cardiaque implantable selon l'invention ;
Figure 2 : un schéma d'un exemple de réalisation du filtre passif de la chaîne électronique de la Figure 1 ;
Figure 3 : un schéma d'un exemple de réalisation de l'amplificateur de la chaîne électronique de la Figure 1 ;
Figure 4 : un diagramme de Bode de l'ensemble constitué par le filtre de la Figure 2 et l'amplificateur de la Figure 3 ;
Figure 5 : un schéma d'un exemple de réalisation du convertisseur analogique-numérique à modulation delta de la chaîne électronique de la Figure 1 ;
Figure 6 : un organigramme général de l'algorithme de détection mis en oeuvre dans le micro-contrôleur de la chaîne électronique de la Figure 1 ;
Figure 7 : un diagramme du rattrapage du signal en fin de période réfractaire absolue (PRA) ;
Figure 8 : un diagramme représentatif :
   - dans sa partie supérieure, d'un signal en sortie d'amplificateur, qui fait suite à une stimulation cardiaque, et
   - dans sa partie inférieure, du signal en sortie du convertisseur analogique-numérique, de rattrapage en fin de PRA ;
Figure 9 : un organigramme du traitement en fin de PRA ;
Figure 10 : un organigramme du traitement de la contrainte de signe selon l'invention ;
Figure 11 un diagramme représentatif d'un signal de diaphonie, suivi d'une contraction ventriculaire spontanée ;
Figure 12 : un diagramme d'un blanking programmé de durée inférieure à 32 ms ;
Figure 13 : un diagramme d'un blanking programmé de durée supérieure à 32 ms ;
Figure 14 : un diagramme d'un blanking automatique ;
Figure 15 : un organigramme du test de diaphonie ;
Figure 16 : un organigramme du traitement du blanking automatique ;
Figure 17 un organigramme de l'algorithme de réglage automatique de la sensibilité.

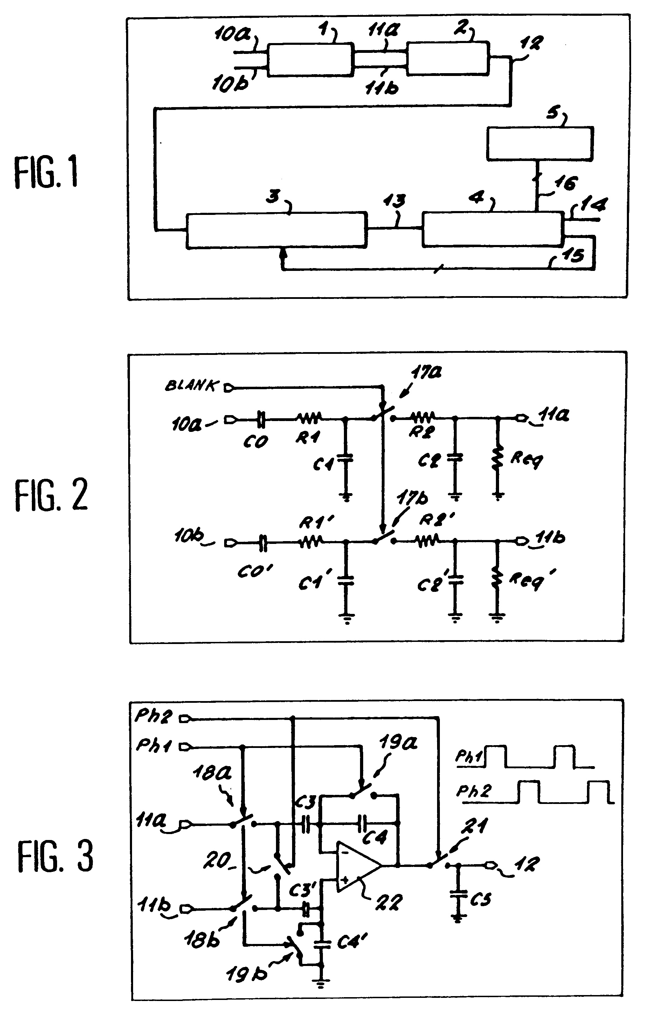

Sur la Figure 1, la chaîne électronique d'acquisition du signal cardiaque comprend essentiellement : un filtre passif à large bande passante 1 ; un amplificateur 2 à capacités commutées, à faible gain ; un convertisseur analogique-numérique 3 à fréquence et pas variables ; un micro-contrôleur 4 réalisant la fonction de détection de l'activité cardiaque et permettant de s'adapter aux phénomènes dus aux stimulations et à la diaphonie, et de mesurer l'amplitude de l'événement détecté ; et un microprocesseur 5 supervisant le fonctionnement du stimulateur cardiaque et assurant notamment la fonction de réglage automatique de sensibilité.

Les références 10a, 10b, 11a, 11b, et 12 à 16 désignent des bornes de liaison électrique, la référence 14 symbolisant la borne de sortie de la chaîne électronique.

La Figure 2 présente un schéma détaillé du filtre 1. Les signaux venant des sondes endocardiaques entrent dans le filtre 1 en 10a et 10b et ressortent en 11a et 11b. L'entrée 10a est connectée à la partie distale de la sonde tandis que l'entrée 10b est connectée soit au boîtier du stimulateur lors d'un recueil monopolaire, soit à la partie proximale de la sonde lors d'un recueil bipolaire. Le commutateur 17a, 17b, commandé par le signal BLANK permet d'isoler la chaîne d'acquisition des sondes afin notamment de protéger les composants électroniques lors des impulsions de stimulation.

Le pôle basse fréquence constitué par la capacité CO (CO') et la résistance d'entrée de l'amplificateur Req (Req') se situe à 0,1 Hz. Le premier pôle haute fréquence constitué par la résistance R1 (R1') et la capacité C1 (C1') se situe à 80 Hz. Le deuxième pôle haute fréquence constitué par la résistance R2 (R2') et la capacité C2 (C2') se situe à 3 kHz. Le fait de ne pas filtrer les basses fréquences présente un double avantage :
- ne pas déformer les signaux de stimulation, d'où une mesure non erronée du paramètre d'efficacité de stimulation,
- ne pas déformer les signaux spontanés basse fréquence (extrasystoles ES, onde T, etc...) afin d'utiliser cette chaîne d'acquisition pour la transmission d'un électrocardiogramme (ECG) endocavitaire.

Le pôle de 80 Hz correspond à la plus haute fréquence des signaux recueillis (ondes P et/ou R). Le pôle à 3kHz constitue une protection contre les interférences électromagnétiques et permet de satisfaire la norme européenne NF EN50 061 sur la sécurité des stimulateurs cardiaques implantables.

La Figure 3 représente un schéma de l'amplificateur 2 à capacités commutées. Ce principe est utilisé pour réduire le nombre des composants électroniques externes, par une intégration de ceux-ci dans la puce électronique, afin de réduire l'encombrement du circuit électronique dans le stimulateur implanté. L'amplificateur 2 à capacités commutées comprend essentiellement un amplificateur différentiel 22, des capacités C3, C3', C4, C4' et C5, et des commutateurs 18a, 18b, 19a, 19b, 20 et 21. Les commutateurs 18a à 19b sont commandés par le signal Ph1 d'une première horloge. Les commutateurs 20 et 21 sont commandés par le signal Ph2 d'une deuxième horloge.

Ces deux horloges distinctes délivrent des signaux Ph1 et Ph2 respectivement, qui ne sont jamais actifs simultanément, comme représenté sur la partie droite de la Figure 3. Pendant la phase active du signal Ph1 de la première horloge, les commutateurs 18a à 19b sont fermés et les commutateurs 20 et 21 sont ouverts. Les capacités C3 et C3' sont chargées chacune à la tension venant du filtre 1, par les bornes 11a et 11b respectivement. Les capacités C4 et C4' sont déchargées.

A la fin de la phase active du signal Ph1, les commutateurs 18a à 19b sont ouverts et les charges des capacités C3 et C3' se répartissent respectivement entre les capacités C3 et C4 d'une part, C3' et C4', d'autre part.

Pendant la phase active du signal Ph2 de la deuxième horloge, les commutateurs 20 et 21 sont fermés. La différence des signaux de tension présents sur les capacités C3 et C3' est amplifiée par l'amplificateur différentiel 22 dans le rapport C3/C4, et le résultat est stocké sur la capacité C5.

Le gain étant réalisé par le rapport de deux capacités, il est possible d'ajuster ce rapport afin de disposer, à la sortie, d'une dynamique maximale.

L'intérêt d'une grande dynamique réside dans le fait que le paramètre d'efficacité de stimulation peut être facilement mesuré. En effet, les signaux de stimulation ne sont déformés ni par un filtrage des basses fréquences en raison de la bande passante du filtre 1, ni par une saturation de l'amplificateur en raison du choix du gain.

A titre d'exemple, en cas de sonde cardiaque auriculaire, le gain peut être fixé à 40 environ et en cas de sonde ventriculaire, le gain peut être fixé à 15 environ.

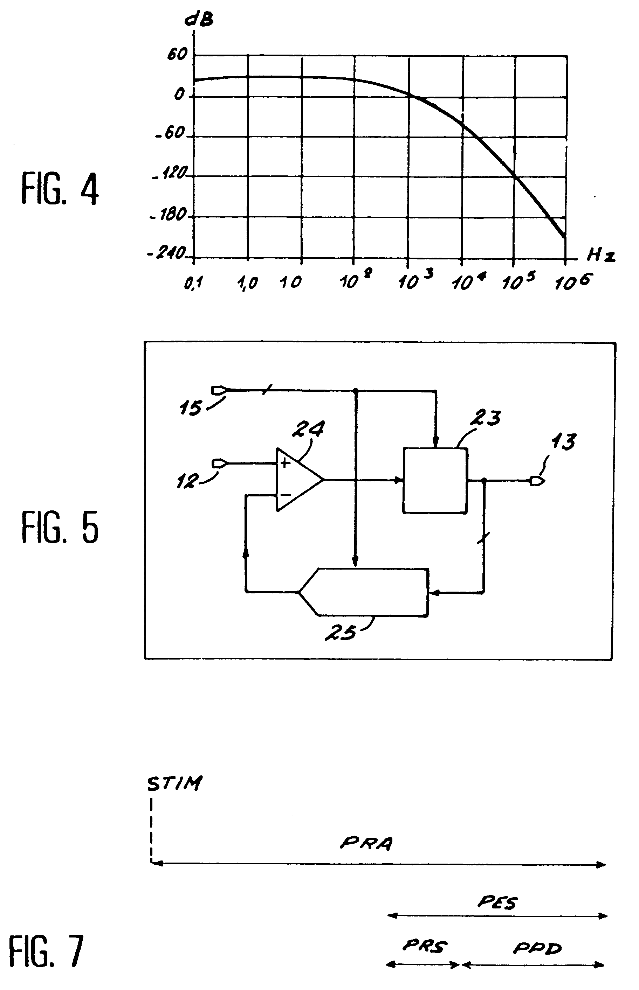

Le gain différentiel de l'ensemble du filtre 1 et de l'amplificateur 2 est représenté sur la Figure 4.

La Figure 5 représente un convertisseur analogique-numérique à modulation delta, comprenant essentiellement un comparateur 24, un compteur 23 et un convertisseur numérique-analogique 25.

Selon le principe de la modulation delta, le résultat d'une différence mesurée (delta) est modulé quelle que soit l'amplitude de la variation du signal d'entrée.

Le principal inconvénient d'un convertisseur A/N à modulation delta est de ne pouvoir suivre correctement un signal présentant une variation rapide d'amplitude du fait que la numérisation se fait par incrémentation ou décrémentation d'un pas de compteur à chaque échantillon.

L'intérêt du montage selon l'invention réside dans le fait que la fréquence et le pas du convertisseur peuvent être modifiés en fonction des variations du signal recueilli. En effet, l'algorithme de détection décrit plus loin renseigne sur les variations d'amplitude subies par le signal venant de la sonde cardiaque. Si le signal venant du myocarde ne présente pas de variations, la conversion est réalisée à une fréquence de base égale par exemple à 1 kHz. Par contre, lorsque la ligne de base du signal présente des variations, dues par exemple à la présence d'un événement ou d'une stimulation, le convertisseur se met à fonctionner à une fréquence plus importante, par exemple 4 kHz, ce qui permet de suivre correctement le signal.

Le pas du convertisseur peut être également modifié en fonction de la sensibilité programmée, c'est-à-dire que le pas d'incrémentation peut être égal non pas à un LSB (bit de poids le plus faible), mais par exemple à 2, 4 ou 8 LSB. Le fait de régler le convertisseur analogique-numérique avec un pas petit, par exemple égal à 1 ou 2 LSB permet d'obtenir une meilleure précision, par contre le fait de régler un pas plus grand permet d'obtenir une plus grande dynamique. Un compromis doit être trouvé entre la sensibilité et la précision que l'on désire obtenir. Dans la réalisation, une table de conversion a été créée afin de fixer le pas correspondant à la fréquence normale et le pas correspondant à la fréquence accélérée pour chaque sensibilité.

L'avantage d'un tel principe est l'adaptation de la fréquence et du pas du convertisseur analogique-numérique en fonction du signal à convertir et, de ce fait, l'adaptation de la consommation en fonction du besoin.

Sur la Figure 5, le signal venant de l'amplificateur 2 et mis à jour chaque milliseconde, arrive par la borne 12, et sort après numérisation par le bus 13. La commande de changement de fréquence de l'opération de comptage, et de changement de pas de comptage, arrive par le bus 15 en provenance du micro-contrôleur 4 qui fonctionne en temps réel, et qui est dédié à la détection. Un algorithme de détection d'événements cardiaques est intégré dans ce micro-contrôleur.

Le principe de l'algorithme est basé sur la recherche d'une variation d'amplitude pendant une durée fixe comprise entre 6 et 12 ms, et égale à 9 ms dans l'exemple décrit. Ce principe est équivalent à un calcul de la pente moyenne sur 9 ms du signal recueilli.

Le principe de calcul de la pente du signal provenant de l'activité cardiaque a déjà été développé par D. W. Davies dans le brevet US 4,905,708 pour distinguer différentes arythmies.

L'algorithme proposé réalise à chaque instant t d'échantillonnage la différence entre la valeur x0 échantillonnée à l'instant t et la valeur x9 échantillonnée à l'instant (t-9). Pour cela, il conserve en mémoire les dix derniers échantillons venant du convertisseur analogique-numérique (CAN) et les décale d'une valeur à chaque instant de saisie t.

La valeur absolue de la différence (x0 - x9) précédemment calculée est comparée à un premier seuil appelé seuil d'accélération. Si ce seuil est dépassé, le CAN se met à fonctionner à la fréquence haute (par exemple 4 kHz, alors que sa fréquence basse de fonctionnement est de 1 kHz par exemple) durant une période prédéfinie, égale à 4 ms dans l'exemple décrit, recyclable à chaque dépassement du seuil d'accélération, et le pas du compteur du CAN peut également être modifié suivant la sensibilité programmée.

La valeur absolue (ABS) de la différence (x0 - x9) est ensuite comparée à un deuxième seuil appelé seuil de détection correspondant à la sensibilité programmée. Si ce second seuil est dépassé, la détection d'un événement est indiquée.

Selon l'invention, pour indiquer la présence d'un événement cardiaque, il est également prévu de rajouter une contrainte sur le signe de la différence (x0 - x9) dans le cas où cette dernière est supérieure au seuil de détection, pour considérer ce dépassement de seuil comme étant dû à la présence d'une contraction spontanée et non à une variation de la ligne de base provoquée par d'autres phénomènes. Cette contrainte de signe est imposée seulement après stimulation, et en cas de diaphonie.

Les modifications de fréquence et de pas du CAN se font par l'intermédiaire du bus 15 (Figure 5). Les seuils de détection et d'accélération sont fournis au micro-contrôleur 4 par le microprocesseur 5. Une détection d'événement est indiquée aux autres parties du stimulateur par l'intermédiaire de la sortie de signal 14 (Figure 1).

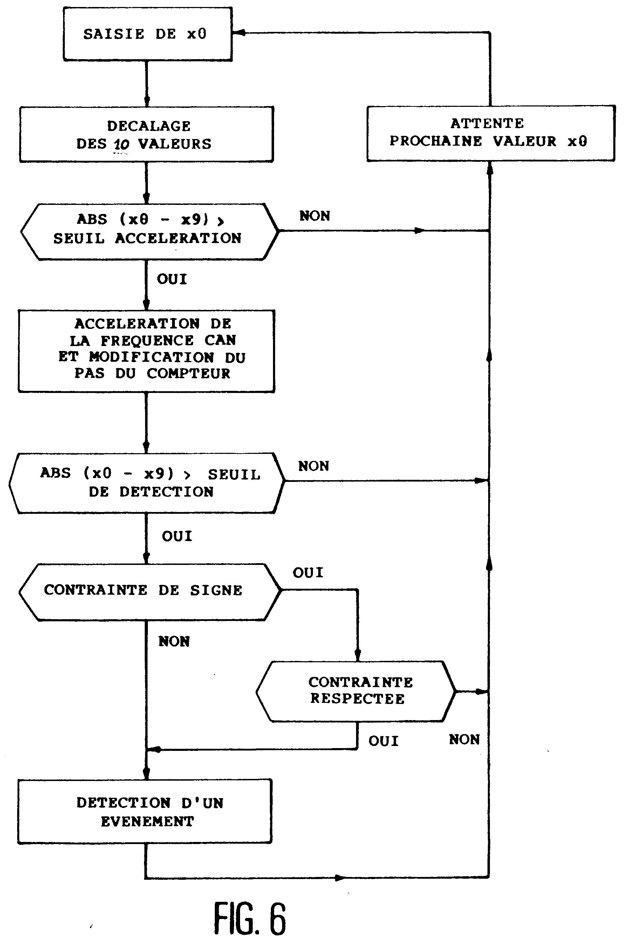

La figure 6 représente un organigramme de l'algorithme principal de détection d'événements cardiaques endocavitaires. En ce qui concerne la contrainte de signe, elle se trouve en service (OUI) ou hors service (NON).

Une phase de rattrapage du signal est prévue après une stimulation.

En cas de stimulation (Stim.), une période réfractaire absolue (PRA) débute dans la chambre stimulée. Cette période dont la durée est réglable par le praticien correspond à la période durant laquelle les cellules du myocarde sont réfractaires à toute stimulation.

Au début de la PRA le convertisseur analogique- numérique (CAN) ainsi que l'algorithme de détection sont arrêtés afin de réduire au maximum la consommation d'énergie.

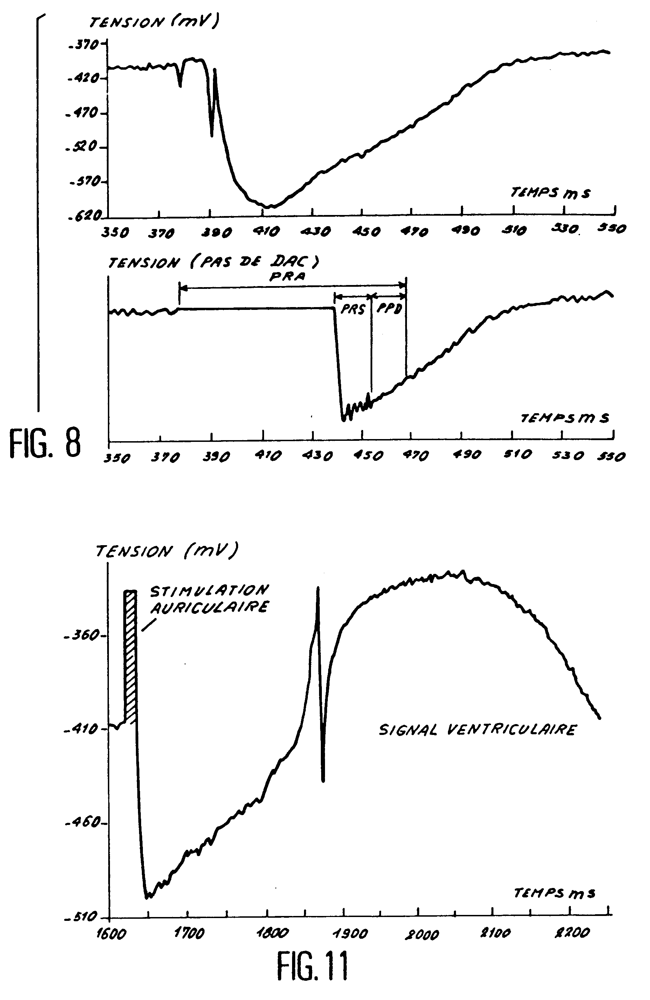

Une autre période appelée période d'étude du signal (PES) est incluse dans la PRA suivant le schéma de la figure 7. La PES se décompose elle-même en deux périodes appelées période de rattrapage du signal (PRS) et période de pré-détection (PPD).

Au début de la PRS, le CAN est remis en marche à la fréquence haute et avec un pas égal à 8 LSB. Ce type de fonctionnement permet de rattraper la variation importante du signal occasionnée par la stimulation entre le début de la PRA et le moment de la réactivation du CAN comme le montre la Figure 8. Dans l'exemple de réalisation décrit, la PRS est fixée à 16 ms, ce qui permet de couvrir l'intégralité de la plage couverte par le CAN.

Après la PRS débute une autre période appelée période de pré-détection (PPD). Durant cette période, la fréquence et le pas du CAN sont de nouveau gérés par l'algorithme de détection, c'est-à-dire qu'il fonctionne à une fréquence normale de 1 kHz avec un pas dit normal et ces paramètres peuvent être modifiés si le signal présente des variations de pente.

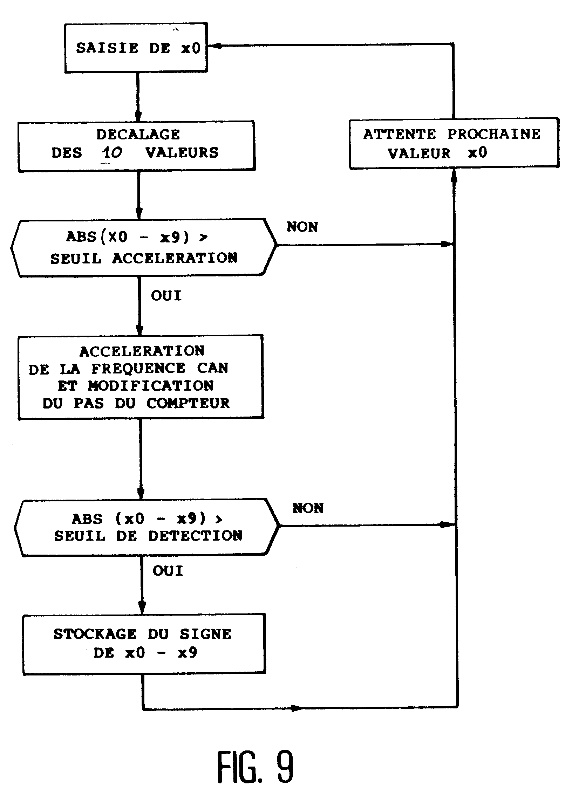

L'algorithme de détection est remis en marche au début de la PPD. Durant cette période, fixée à 32 ms dans l'exemple de réalisation décrit, si une ou plusieurs détections surviennent, elles sont considérées comme étant provoquées par une variation de la ligne de base du signal cardiaque due à la stimulation. Le signe de la dernière différence (x0 - x9) qui a engendré une détection durant la PPD est stocké. L'organigramme de la figure 9 représente cette partie du traitement.

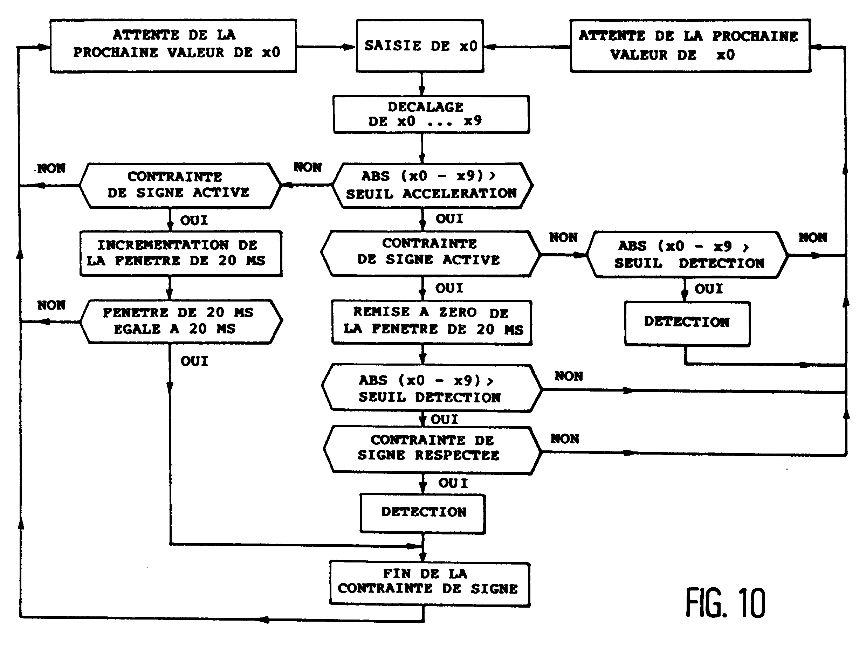

Après la fin de la PRA, une condition supplémentaire, la contrainte de signe, est ajoutée pour considérer une détection comme étant réellement provoquée par un événement. Cette condition est un test du signe de la différence (x0 - x9). Seules les différences (x0 - x9) dont le signe est contraire à celui stocké et dont la valeur absolue de l'amplitude est supérieure au seuil de détection sont considérées comme étant provoquées par un événement cardiaque. Ce test de signe est décrit par l'organigramme de la Figure 10.

Cette contrainte sur le signe de la pente calculée est supprimée lorsque :
- la valeur absolue de la différence (x0 - x9) ne dépasse pas le seuil d'accélération durant une certaine période, fixée à 20 ms dans l'exemple décrit (Figure 10). Cette dernière condition indique que la ligne de base n'est pratiquement plus perturbée,
- la valeur absolue de la différence (x0 - x9) dépasse le seuil de détection, avec un signe de la différence (x0 - x9) contraire au signe stocké, ce qui correspond à la détection d'un événement cardiaque,
- 400 ms après la stimulation.

Un traitement du phénomène de diaphonie est prévu.

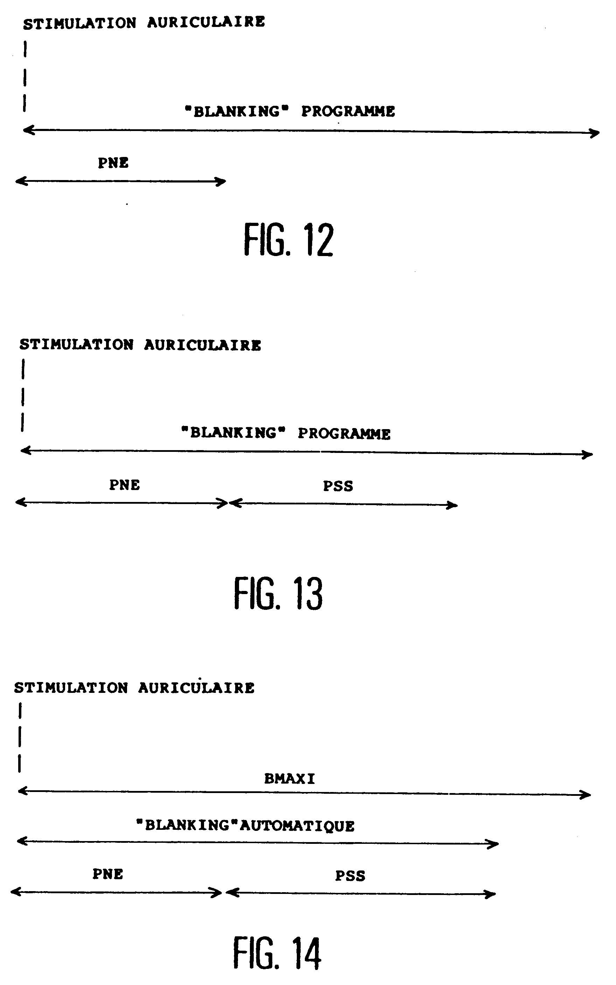

En cas de stimulation auriculaire (ventriculaire), le signal ventriculaire (auriculaire) peut présenter une déflexion due au phénomène de diaphonie. La figure 11 illustre ce phénomène sur le signal ventriculaire lors d'une stimulation auriculaire, à l'exclusion de la contraction ventriculaire spontanée induite par la stimulation auriculaire, et représentée entre les temps 1850 et 1900, le signal ventriculaire correspond à la diaphonie. Cette déflexion peut être plus ou moins importante en fonction de l'énergie de stimulation auriculaire, des caractéristiques des sondes (matériau, surface) et du mode de stimulation et de recueil (monopolaire, bipolaire).

Ce phénomène de diaphonie est décrit d'une manière générale dans la littérature par C. D. Johnson dans "Atrial Synchronous Ventricular Inhibited (VDD) Pacemaker-Mediated Arrhythmia Due to Atrial Undersensing and Atrial Lead Oversensing of Far-Field Ventricular Afterpotentials of Paced Beats : Cross-talk", Pace, Vol 9, pp 710-719, et d'une manière plus particulière par W. J. Combs et al. dans "Cross-Talk in Bipolar Pacemakers", Pace, Vol 12, pp 1613 - 1621.

Lors d'une stimulation auriculaire, une période appelée "blanking" débute. La durée du "blanking" est traditionnellement programmée par le praticien en fonction de la diaphonie observée sur un ECG endocavitaire.

L'invention présente l'avantage de fonctionner soit avec une durée de "blanking" programmée, soit avec une durée de "blanking" s'ajustant automatiquement en fonction de l'importance de la diaphonie présente sur le signal.

Le brevet U.S. No. 4,974,589 décrit une méthode de réglage automatique de la période de "blanking" par redéclenchement d'une période d'écoute à chaque détection ventriculaire après une stimulation auriculaire. La période de "blanking" est considérée comme terminée si aucune détection n'est enregistrée durant la période d'écoute, avec toutefois une valeur maximale de la durée de "blanking" à ne pas dépasser, par exemple de 50 ms.

Quel que soit le type de "blanking" choisi (programmé ou automatique), l'algorithme de détection stocke la valeur de la ligne de base (VLB) au moment de la stimulation, et s'arrête. Débute alors une première période appelée période de non écoute (PNE) durant laquelle la chaîne de détection ventriculaire du stimulateur cardiaque est déconnectée des sondes (le signal BLANK est actif (Figure 2)). Dans l'exemple de réalisation décrit, la durée de cette période est fixée à 14 ms. Cette technique est très couramment utilisée pour protéger les composants électroniques placés à l'entrée de la chaîne d'acquisition contre les phénomènes de surtension dus à la stimulation.

Pour la suite, il est nécessaire de différencier le "blanking" programmé et le "blanking" automatique. Pour une durée de "blanking" programmée par le médecin, il existe encore deux types de traitement possible :

Pour une durée de "blanking" programmée courte, inférieure à 32 ms dans l'exemple de réalisation décrit, le CAN se met à fonctionner, à la fin de la PNE, à la fréquence haute avec un pas correspondant à la sensibilité programmée et ce, jusqu'à la fin de la période de "blanking" (Figure 12).

Pour une durée de "blanking" programmée supérieure à 32 ms, le CAN se met à fonctionner à la fin de la PNE, à la fréquence haute avec un pas égal à 8 LSB et ce, durant une période de 16 ms appelée période de suivi du signal (PSS). Cette solution est adoptée car lorsque le praticien programme une durée de "blanking" importante, cela signifie que la déflexion observée l'est également. Par ce biais, il est possible de suivre correctement une variation importante et rapide du signal (Figure 13).

Pour une durée de "blanking" réglée automatiquement, le CAN se met à fonctionner à la fin de la PNE à la fréquence haute avec un pas égal à 8 LSB. L'algorithme de détection est également réactivé à la fin de la PNE. A l'issue de la PNE, débute une période appelée période de suivi de signal (PPS) durant laquelle l'algorithme effectue un test de fin de "blanking". Une fois ce test validé, la période de "blanking" est terminée (Figure 14). Le test de fin de "blanking" est présenté par la suite.

Lors d'un réglage manuel de la durée du "blanking", l'algorithme de détection est remis en marche à la fin du "blanking" par une commande venant du microprocesseur, il commence par saisir la valeur présente à la sortie du CAN, nommée valeur de fin de "blanking" (VFB), et il effectue un test de diaphonie qui consiste à mesurer l'amplitude et le signe de la déflexion en fin de "blanking". Cette amplitude est calculée par la différence entre la valeur VFB et la valeur VLB :
- Si la valeur absolue de l'amplitude de la déflexion due à la stimulation auriculaire est inférieure au seuil de détection programmé, alors la détection s'effectue normalement sans contrainte de signe ;
- Si la valeur absolue de cette amplitude est supérieure au seuil de détection programmé, il y a risque de détecter un signal occasionné par la déflexion. Pour cela, une détection d'événement ne sera indiquée à l'instant t que si la valeur de la différence (x0 - x9) calculée à l'instant t est supérieure au seuil, et si le signe de cette différence est le même que celui de la déflexion.

Cette contrainte sur le signe de la pente calculée est enlevée lorsque durant une période égale par exemple à 20 ms, la valeur absolue de la différence (x0 - x9), soit ABS (x0 - x9) ne dépasse pas le seuil d'accélération (Figure 10). Cette dernière condition indique que la ligne de base n'est pratiquement plus perturbée.

Cette solution sous-entend naturellement que la période de "blanking" couvre la première pente de la déflexion.

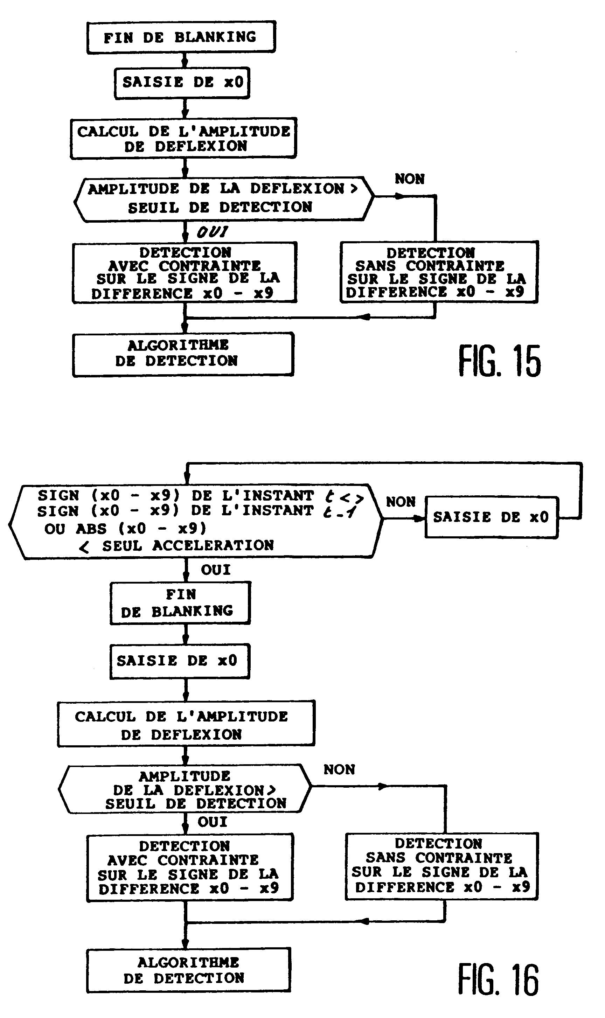

Lors d'un réglage automatique de la durée du "blanking", le test de fin de "blanking" repose sur le double critère d'inversion de pente moyenne (x0 - x9), ou de pente moyenne (x0 -x9) devenant très faible. En effet, si les signes de deux différences (x0 -x9) consécutives sont différents ou si la valeur absolue de la différence (x0 - x9) devient inférieure au seuil d'accélération on peut considérer que le signal présente sa déflexion maximale (Figure 16). Une fois cette inversion trouvée, l'algorithme de détection considère la période de "blanking" comme terminée et il réalise le même test de diaphonie que décrit précédemment (Figure 15).

Une période maximale durant laquelle l'inversion doit survenir est préalablement fixée afin d'éviter de rester trop longtemps en période de "blanking" si le signal présente une anomalie. Cette période est nommée "blanking" maximum BMAXI (Figure 14).

Comme précédemment décrit, le principe de détection d'un événement cardiaque repose sur la comparaison entre la pente moyenne du signal sur une durée de 6 à 12 ms, égale de préférence à 9 ms et une valeur réglable par le praticien appelée seuil de détection.

De nombreuses études montrent que l'amplitude et la forme des ondes P et R varient dans le temps en fonction notamment de l'activité physique, du stress, de l'évolution de la pathologie, etc... De ce fait, la sensibilité réglée par la praticien le jour de l'implantation peut s'avérer être soit trop faible dans certains cas, d'où une absence de détection de l'activité spontanée, soit trop importante lors de la présence de bruits, d'où une détection permanente. Dans les deux cas, le stimulateur va envoyer une impulsion de stimulation à la fin de chaque cycle d'écoute.

A l'encontre d'une perte de détection, la solution consiste à mettre en oeuvre un réglage automatique de la sensibilité. Dans ce domaine deux voies sont à considérer :

Les brevets US 4,768,511 et US 4,766,902 traitent d'un réglage automatique de sensibilité par comparaison de l'amplitude du signal recueilli avec deux seuils. Dans le cadre de l'invention, la sensibilité est considérée comme bien réglée si le premier seuil est dépassé et pas le second, la sensibilité est trop importante si les deux seuils sont dépassés, et la sensibilité est trop faible si aucun des deux seuils n'est dépassé. Le seuil de détection est donc modifié suivant les conditions précédemment énoncées.

Dans les brevets EP 0 349 130, et US 4,827,934 le même principe est utilisé pour la recherche de la bonne sensibilité, mais en cas de correction de la sensibilité, ce n'est pas le seuil de détection qui est modifié mais le gain de la chaîne de détection.

Dans le brevet US 4,708,144, la recherche de sensibilité est réalisée suivant une méthode qui consiste à rechercher la valeur maximale de l'amplitude du signal à détecter, et à réaliser une moyenne avec la valeur maximale de l'événement précédent. La sensibilité est modifiée par un changement de gain de la chaîne de détection.

Le principe retenu consiste à rechercher la valeur maximale de la valeur absolue de la différence (x0 - x9) lors de la détection d'un événement, et à calculer la moyenne des valeurs maximales de plusieurs événements précédemment détectés pour fixer la nouvelle sensibilité.

Pour obtenir cette valeur maximale, lors de la détection d'un événement, une période de recherche de maximum (PRM), fixée dans l'exemple de réalisation décrit à 48 ms, est lancée, durant laquelle la valeur absolue de la différence (x0 - x9) est comparée avec une valeur appelée MAX initialisée à zéro au début de la PRM. Si la valeur absolue de la différence (x0 - x9) est plus grande que la valeur MAX, alors MAX prend la valeur de la valeur absolue de la différence (x0 - x9). Cette opération est réalisée par le micro-contrôleur 4 dédié à la détection.

A la fin de la PRM, le micro-processeur 5 qui supervise l'ensemble du fonctionnement du stimulateur cardiaque vient lire la valeur MAX par le bus 16 (Figure 1) et il calcule la moyenne des huit dernières valeurs MAX stockées en mémoire.

Le micro-processeur 5 calcule un seuil intermédiaire fixé à un certain pourcentage de la moyenne des huit valeurs MAX. Dans l'exemple de réalisation décrit, le pourcentage retenu est de 37,5%.

Le micro-processeur 5 possède en mémoire une table dans laquelle se trouvent toutes les sensibilités qu'il est possible de programmer ainsi que les seuils de détection, les seuils d'accélération, les fréquences et les pas du CAN correspondants.

Si le seuil intermédiaire est plus petit que le seuil de détection programmé, alors le nouveau seuil de détection est celui directement inférieur dans la table au seuil de détection programmé.

Si le seuil intermédiaire est plus grand que le seuil de détection directement supérieur dans la table au seuil de détection programmé, alors le nouveau seuil de détection est le seuil supérieur dans la table au seuil programmé.

A l'initialisation, la sensibilité initiale est programmable par la praticien. Par défaut, ses valeurs sont 1.0 mV dans l'oreillette de 2.2 mV dans le ventricule. Les huit valeurs MAX sont initialisées à la sensibilité programmée divisée par 0.375.

En cas de stimulation, la valeur MAX qui est retenue correspond à la valeur MAX initiale, soit la sensibilité initiale divisée par le pourcentage retenu qui est de 0.375 dans notre réalisation. Suivant ce principe au bout de huit stimulations consécutives, la sensibilité programmée a pour valeur la sensibilité initiale.

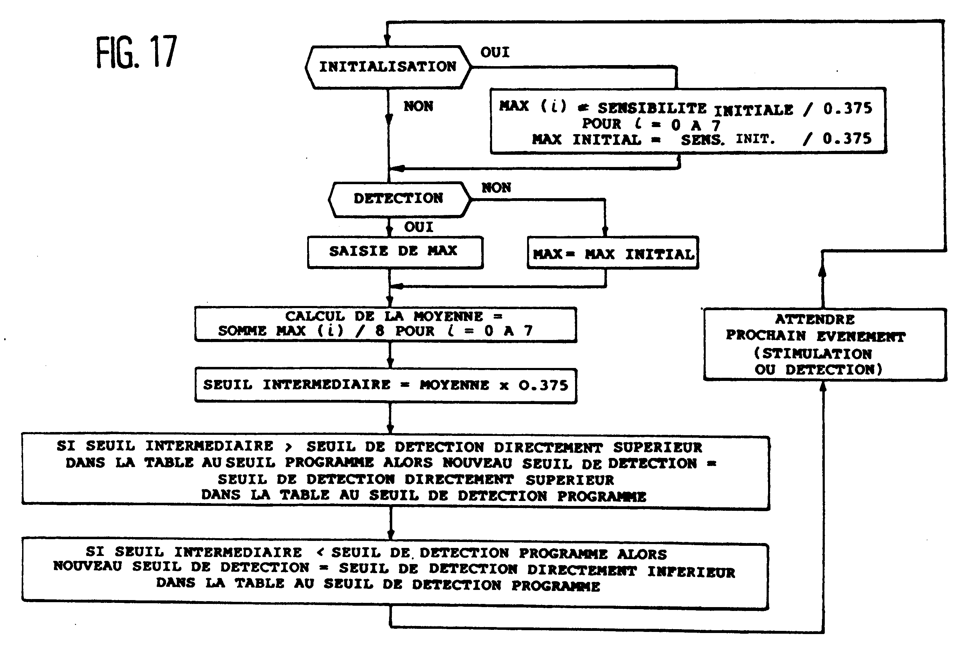

L'organigramme de l'algorithme de réglage automatique de la sensibilité est décrit figure 17.

L'un des avantages de la présente invention consiste en une réduction du nombre des composants électroniques externes et en une plus grande intégration de composants dans la puce grâce aux capacités commutées de l'amplificateur.

Un autre avantage, lié à la large bande passante du filtre et à la grande dynamique de l'amplificateur est qu'une chaîne électronique unique d'acquisition et de détection de l'activité cardiaque est utilisée à la fois pour le fonctionnement du stimulateur cardiaque et pour l'écoute de l'ECG en télémétrie.

Un autre avantage encore de l'invention est de proposer un algorithme de détection plus adaptatif.

Ces avantages proviennent du fait que le filtre à large bande et l'amplificateur à grande dynamique fournissent un signal cardiaque non déformé, qui est numérisé et qui fait ensuite seulement l'objet d'un traitement.

## Revendications

1. Stimulateur cardiaque implantable, dans lequel la chaîne électronique d'acquisition et de détection de l'activité cardiaque endocavitaire comporte un filtre passif, un amplificateur à capacités commutées, un convertisseur analogique-numérique, un microprocesseur, et un algorithme pour assurer la détection des événements cardiaques spontanés, caractérisé en ce que :
- le filtre (1) présente une bande passante s'étendant de 0,1 à 80 Hz environ ;
- l'amplificateur (2) présente à la sortie une dynamique de 0,8 V environ et un gain inférieur à 50 ;
- le convertisseur analogique-numérique (3) est à modulation delta, à fréquence et à pas variables et adaptés en fonction du signal à convertir.

2. Stimulateur selon la revendication 1 caractérisé en ce que le convertisseur anologique-numérique fonctionne à deux fréquences, l'une d'environ 1 kHz, l'autre d'environ 4 kHz.

3. Stimulateur cardiaque selon la revendication 1 caractérisé en ce que la chaîne électronique d'acquisition et de détection de l'activité cardiaque présente une bande passante large, un gain faible et une dynamique grande pour assurer la détection des événements spontanés auriculaires et/ou ventriculaires, la mesure du paramètre d'efficacité de stimulation, et la transmission d'un ECG endocavitaire par télémétrie.

4. Stimulateur selon la revendication 1, caractérisé en ce qu'il comporte un réglage automatique de la période de "blanking".

5. Stimulateur selon la revendication 1, caractérisé en ce qu'il comporte un réglage automatique de la sensibilité.

6. Stimulateur selon la revendication 1, caractérisé en ce que la commande de changement de fréquence et de changement de pas du convertisseur analogique-numérique est réalisée par comparaison de la valeur absolue de la variation d'amplitude du signal recueilli pendant une durée de 6 à 12 ms et de préférence de 9 ms avec un seuil dénommé seuil d'accélération.

7. Stimulateur selon la revendication 1, caractérisé en ce que la détection d'un événement cardiaque spontané auriculaire ou ventriculaire est réalisée par une comparaison de la valeur absolue de la variation d'amplitude du signal recueilli pendant une durée de 6 à 12 ms et de préférence de 9 ms avec un seuil appelé seuil de détection.

8. Stimulateur selon la revendication 1, caractérisé en ce que la fonction de détection d'un événement cardiaque auriculaire ou ventriculaire est réalisée par traitement numérique avec un microcontrôleur dédié.

9. Stimulateur selon la revendication 7, caractérisé en ce que le signe d'une variation d'amplitude du signal recueilli pendant une durée de 6 à 12 ms et de préférence de 9 ms avant la fin de la période réfractaire absolue (PRA) supérieure au seuil de détection étant mémorisé, la détection après la PRA, d'un événement spontané n'est indiquée que si la valeur absolue de la variation d'amplitude du signal recueilli pendant une durée de 6 à 12 ms et de préférence de 9 ms est supérieure au seuil de détection et si le signe de cette variation est contraire à celui mémorisé durant la PRA.

10. Stimulateur selon la revendication 1, caractérisé en ce que la durée de "blanking" ventriculaire est réglable manuellement ou automatiquement, et dans le cas d'un réglage automatique, la durée de "blanking" est ajustée à chaque stimulation auriculaire en fonction de l'amplitude de la diaphonie.

11. Stimulateur selon la revendication 10, caractérisé en ce qu'au cours d'un réglage automatique de la durée du "blanking", la fin de celui-ci est obtenue lorsque le signe s'inverse entre deux variations consécutives du signal recueilli pendant une durée de 6 à 12 ms et de préférence de 9 ms.

12. Stimulateur selon la revendication 10, caractérisé en ce que l'amplitude de la déflexion du signal ventriculaire consécutive à une stimulation auriculaire est mesurée à la fin de la période de "blanking" et le signe de cette déflexion est mémorisé, et si l'amplitude de cette déflexion est supérieure au seuil de détection, à l'issue de la période de "blanking" seules les valeurs absolues des variations d'amplitude du signal ventriculaire recueilli pendant une durée de 6 à 12 ms et de préférence de 9 ms supérieures au seuil de détection et dont le signe est identique à celui de la déflexion seront considérées comme étant dues à la présence d'un événement ventriculaire, et dans le cas contraire, la détection est réalisée sans contrainte de signe.

13. Stimulateur selon la revendication 1, caractérisé en ce que lors d'un réglage automatique de sensibilité, une recherche de la valeur absolue de la variation d'amplitude maximale du signal recueilli pendant une durée de 6 à 12 ms et de préférence de 9 ms débute sur une détection d'un événement cardiaque spontané auriculaire et/ou ventriculaire et se prolonge durant une période égale de préférence à 48 ms.

14. Stimulateur selon la revendication 1, caractérisé en ce que lors d'un réglage automatique de la sensibilité, la sensibilité initiale est programmable et cette valeur multipliée de préférence par 0.375, remplace en cas de stimulation la variation d'amplitude maximale recherchée après une détection selon la revendication 13.

15. Stimulateur selon la revendication 1, caractérisé en ce que lors d'un réglage automatique de la sensibilité le nouveau seuil de détection est fixé à un certain pourcentage, de préférence 37,5%, de la moyenne des huit dernières valeurs maximales de la variation du signal sur 9 ms recueilli à chaque détection et stimulation.

## Claims

1. An implantable cardiac pacemarker in which an electronic circuit for the acquisition and detection of the intracardial activity comprises a passive filter, an amplifier with switched capacitors, an analog to digital converter, a microprocessor, and an algorithm to insure the detection of spontaneous cardiac events, characterized in that :
- the filter (1) has a pass range spreading from 0.1 to 80 Hz approximately;
- the amplifier (2) presents to the output a dynamic rage of 0.8 V approximately and a gain lower than 50;
- the analog-to-digital converter (3) has a delta modulation, with a variable sampling frequency and a variable step magnitude, which are adapted according to the signal to be converted.

2. The pacemaker of claim 1, characterized in that the analog-to-digital converter functions at two frequencies, one of approximately 1 kHz, the other of approximately 4 kHz.

3. The pacemaker of claim 1, characterized in that the electronic acquisition and detection circuit for the intracardial activity, has a large bandwidth, a low gain and a large dynamic range, to provide for the detection of spontaneous atrial and/or ventricular events, the measure of the efficiency parameter of the stimulation, and the transmission of an intracardial ECG by telemetry.

4. The pacemaker of claim 1, characterized in that the pacemaker includes an automatic adjustment of the period of blanking,

5. The pacemaker of claim 1, characterized in that the pacemaker includes an automatic adjustment of the sensivity.

6. The pacemaker of claim 1, characterized in that the command of the frequency change and of the step change of the analog-to-digital converter is realized by comparison of the absolute value of the variation of amplitude of the signal collected during a duration of from 6 to 12 ms, and preferably 9 ms, with a threshold called threshold of acceleration.

7. The pacemaker of claim 1, characterized in that the detection of an atrial or ventricular spontaneous cardiac event is realised by a comparison of the absolute value of the variation of amplitude of the signal collected during a duration of 6 to 12 ms, and preferably 9 ms, with a threshold called threshold of detection.

8. The pacemaker of claim 1, characterized in that the function of detection of a ventricular or atrial cardiac event is realized by numerical processing with a dedicated microcontroller.

9. The pacemaker of claim 7, characterized in that, the sign of a variation of amplitude of the signal collected during a duration of 6 to 12 ms, and preferably 9 ms, before the end of the absolute refractory period (ARP), where the absolute value of the amplitude variation is greater than the threshold of detection, being memorized, the detection, after the ARP, of a spontaneous event is then indicated only if the absolute value of the variation of amplitude of the signal collected during a duration of 6 to 12 ms, and preferably 9 ms, is greater than the threshold of detection, and if the sign of this amplitude variation is of opposite sign to the sign memorized during the ARP.

10. The pacemaker of claim 1, characterized in that the duration of the ventricular blanking is adjustable manually or automatically, and in the case of an automatic adjustment, the duration of blanking is adjusted at each atrial stimulation according to the amplitude of the cross-talk.

11. The pacemaker of claim 10, characterized in that, in the course of an automatic adjustment of the blanking duration, the end of the blanking duration is determined when the sign inverts between two consecutive variations of the signal collected during a duration of from 6 to 12 ms, and preferably 9 ms.

12. The pacemaker of claim 10, characterized in that the amplitude of the deflection of the ventricular signal following an atrial stimulation is measured at the end of the blanking period, and the sign of this deflection is memorized, and if the amplitude of this deflection is greater than the threshold of detection, at the end of the blanking period only the absolute values of the amplitude variations of the ventricular signal collected during a duration of 6 to 12 ms, preferably 9 ms, greater than the threshold of detection, and the sign of which is identical to the deflection sign, will be considered as due to the presence of a ventricular event, and in the contrary case, the detection is realized without the constraint of sign.

13. The pacemaker of claim 1, characterized in that, during an automatic sensitivity adjustment, a search of the absolute value of the maximal amplitude variation of the signal collected during a duration of 6 to 12 ms, and preferably 9 ms, begins on a detection of a spontaneous atrial and/or ventricular cardiac event, and extends over a given period, preferably 48 ms.

14. The pacemaker of claim 1, characterized in that, during an automatic adjustment of the sensivity, the initial sensivity is programmable and this value multiplied preferably by 0.375 is used, in the case of a stimulation, in place of the maximal amplitude variation searched after a detection according to claim 13.

15. The pacemaker of claim 1, characterized in that, during an automatic adjustment of the sensivity, the new threshold of detection is set at a certain percentage preferably 37.5%, of the average of the last eight maximal values of the variation of the signal during 9 ms, collected for each detection and stimulation.

## Patentansprüche

1. Implantierbarer Herzschrittmacher, bei dem die elektronische Kette der Akquisition und Erfassung der endokavitären Herztätigkeit ein passives Filter, einen Verstärker mit geschalteten Kapazitäten, einen Analog-Digital-Wandler, einen Mikropozessor und einen Algorithmus umfaßt, um die Erfassung spontaner Herz-Ereignisse sicherzustellen,
**dadurch gekennzeichnet, daß**
- das Filter (1) ein Paßband aufweist, das sich etwa von 0,1 bis 80 Hz erstreckt;
- der Verstärker (2) an seinem Ausgang eine Dynamik von etwa 0,8 V und eine Verstärkung kleiner 50 aufweist ;
- der Analog-Digital-Wandler (3) mit Deltamodulation arbeitet, wobei die Frequenz und Schrittweite variabel sind und in Abhängigkeit des zu wandelnden Signals angepaßt sind.

2. Herzschrittmacher nach Anspruch 1,
**dadurch gekennzeichnet, daß**
der Analog-Digital-Wandler mit zwei Frequenzen arbeitet, wobei die eine bei etwa 1 kHz und die andere bei etwa 4 kHz liegt.

3. Herzschrittmacher nach Anspruch 1,
**dadurch gekennzeichnet, daß**
die elektronische Kette der Akquisition und der Erfassung der Herztätigkeit ein breites Paßband, eine geringe Verstärkung und eine große Dynamik aufweist, um die Erfassung der spontanen aurikulären und/oder ventrikulären Ereignisse, die Messung des Parameters der Stimulationswirksamkeit und die Übertragung eines endokavitären Elektrokardiogramms (EKG) mittels Telemetrie sicherzustellen.

4. Herzschrittmacher nach Anspruch 1,
**dadurch gekennzeichnet, daß**
er mit einer automatischen Regelung der "Blanking"-Periode versehen ist.

5. Herzschrittmacher nach Anspruch 1,
**dadurch gekennzeichnet, daß**
er mit einer automatischen Empfindlichkeitsregelung versehen ist.

6. Herzschrittmacher nach Anspruch 1,
**dadurch gekennzeichnet, daß**
die Steuerung des Frequenzwechsels und des Schrittwechsels des Analog-Digital-Wandlers durch einen Vergleich des Absolutwerts der Amplitudenänderung des aufgenommenen Signals während einer Dauer von 6 bis 12 ms, vorzugsweise 9 ms, mit einer als Beschleunigungsschwelle bezeichneten Schwelle erfolgt.

7. Herzschrittmacher nach Anspruch 1,
**dadurch gekennzeichnet, daß**
die Erfassung eines spontanen aurikulären oder ventrikulären Herz-Ereignissses mittels eines Vergleichs des Absolutwerts der Amplitudenänderung des aufgenommenen Signals während einer Dauer von 6 bis 12 ms, vorzugsweise 9 ms, mit einer Erfassungsschwelle genannten Schwelle erfolgt.

8. Herzschrittmacher nach Anspruch 1,
**dadurch gekennzeichnet, daß**
die Erfassungsfunktion eines aurikulären oder ventrikulären Herz-Ereignisses mittels digitaler Verarbeitung durch einen entsprechenden Mikrocontroller realisiert ist.

9. Herzschrittmacher nach Anspruch 7,
**dadurch gekennzeichnet, daß**
das Vorzeichen einer Amplitudenänderung des während einer Dauer von 6 bis 12 ms, vorzugsweise 9 ms, vor dem Ende der absoluten Refraktärphase (PRA) aufgenommenen Signals, die größer als die Erfassungsschwelle ist, gespeichert ist, und die Erfassung eines spontanen Ereignisses nach der PRA nur angezeigt wird, wenn der Absolutwert der Amplitudenänderung des während einer Dauer von 6 bis 12 ms, vorzugsweise 9 ms, aufgenommenen Signals größer als die Erfassungsschwelle ist und das Vorzeichen dieser Änderung entgegengesetzt zu dem während der PRA gespeicherten ist.

10. Herzschrittmacher nach Anspruch 1,
**dadurch gekennzeichnet, daß**
die Dauer des ventrikulären "Blanking" manuell oder automatisch regelbar ist, und im Fall einer automatischen Regelung die Dauer des "Blanking" bei jeder aurikulären Stimulation als Funktion der Amplitude des Nebensprechens eingestellt wird.

11. Herzschrittmacher nach Anspruch 10,
**dadurch gekennzeichnet, daß**
im Verlauf einer automatischen Regelung der Dauer des "Blanking" das Ende desselben erhalten wird, wenn das Vorzeichen sich zwischen zwei aufeinanderfolgenden Änderungen des während einer Dauer von 6 bis 12 ms, vorzugsweise 9 ms, aufgenommenen Signals umkehrt.

12. Herzschrittmacher nach Anspruch 10,
**dadurch gekennzeichnet, daß**
die Amplitude der Auslenkung des auf eine aurikuläre Stimulation folgenden ventrikulären Signals am Ende der "Blanking"-Periode gemessen wird, und das Vorzeichen dieser Auslenkung gespeichert wird und, wenn die Amplitude dieser Auslenkung größer als die Erfassungsschwelle ist, am Ende der "Blanking"-Periode lediglich die Absolutwerte der Amplitudenänderungen des während einer Dauer von 6 bis 12 ms, vorzugsweise 9 ms, aufgenommenen ventrikulären Signals, die größer als die Erfassungsschwelle sind, und deren Vorzeichen identisch mit dem der Auslenkung ist, als solche berücksichtigt werden, die durch das Vorliegen eines ventrikulären Ereignisses bedingt sind, und im gegensätzlichen Fall die Erfassung ohne Beschränkung des Vorzeichens realisiert wird.

13. Herzschrittmacher nach Anspruch 1,
**dadurch gekennzeichnet, daß**
bei einer automatischen Empfindlichkeitsregelung eine Suche nach dem Absolutwert der maximalen Amplitudenänderung des während einer Dauer von 6 bis 12 ms, vorzugsweise 9 ms, aufgenommenen Signals auf eine Erfassung eines spontanen aurikulären und/oder ventrikulären Herz-Ereignisses hin beginnt, und sich für eine Dauer von vorzugsweise 48 ms fortsetzt.

14. Herzschrittmacher nach Anspruch 1,
**dadurch gekennzeichnet, daß**
bei einer automatischen Empfindlichkeitsregelung die anfängliche Empfindlichkeit programmierbar ist, wobei dieser vorzugsweise mit 0,375 multiplizierte Wert im Fall der Stimulation die gesuchte maximale Amplitudenänderung nach einer Erfassung gemäß Anspruch 13 ersetzt.

15. Herzschrittmacher nach Anspruch 1,
**dadurch gekennzeichnet, daß**
bei einer automatischen Empfindlichkeitsregelung die neue Erfassungsschwelle auf einen gewissen Prozentsatz von vorzugsweise 37,5% des Mittelwerts der acht letzten maximalen Werte der Änderung des bei jeder Erfassung und Stimulation aufgenommenen Signals über 9 ms festgelegt wird.
